# EUROPEAN PATENT SPECIFICATION

(11) **EP 3 893 896 B1**
(45) Date of publication and mention of the grant of the patent: **19.11.2025**
(21) Application number: 19832854.4
(22) Date of filing: 13.12.2019
(51) Int. Cl.: A61K 35/02, A61P 11/00

(54) **BARN DUST EXTRACT FOR USE IN THE TREATMENT OF ALLERGIC DISEASES**
STALLSTAUBANDEXTRAKT ZUR VERWENDUNG BEI DER BEHANDLUNG VON ALLERGISCHEN KRANKHEITEN
EXTRAIT DE POUSSIÈRE D'ÉTABLE POUR UTILISATION DANS LE TRAITEMENT DES MALADIES ALLERGIQUES

(30) Priority: 14.12.2018 LU 101064
(43) Date of publication of application: 20.10.2021
(73) Proprietor: Helmholtz Zentrum München Deutsches Forschungszentrum für Gesundheit und Umwelt GmbH, 85764 Neuherberg (DE); Arizona Board of Regents on behalf of the University of Arizona, Tuscon, AZ 85721 (US)
(72) Inventor: VON MUTIUS, Erika, 80805 München (DE); BRACHER, Franz, 81377 München (DE); MÜLLER, Christoph, 81377 München (DE); VERCELLI, Donata, Tucson, Arizona 85718 (US)
(74) Representative: Schiweck Weinzierl Koch Patentanwälte Partnerschaft mbB
(86) International application number: PCT/EP2019/085016
(87) International publication number: WO 2020/120724

(56) References cited:
- US-A1- 2008 112 983
- PETERS M ET AL: "Arabinogalactan isolated from cowshed dust extract protects mice from allergic airway inflammation and sensitization", JOURNAL OF ALLERGY AND CLINICAL IMMUNOLOGY, ELSEVIER, AMSTERDAM, NL, vol. 126, no. 3, 1 September 2010 (2010-09-01), pages 648 - 656.e4, XP027399158, ISSN: 0091-6749, [retrieved on 20100909]
- M. J. SCHUIJS ET AL: "Farm dust and endotoxin protect against allergy through A20 induction in lung epithelial cells", SCIENCE, vol. 349, no. 6252, 4 September 2015 (2015-09-04), US, pages 1106 - 1110, XP055601415, ISSN: 0036-8075, DOI: 10.1126/science.aac6623
- OBER CAROLE ET AL: "Immune development and environment: lessons from Amish and Hutterite children", CURRENT OPINION IN IMMUNOLOGY, vol. 48, 29 August 2017 (2017-08-29), pages 51 - 60, XP085259599, ISSN: 0952-7915, DOI: 10.1016/J.COI.2017.08.003
- GOZDZ: "Innate Immunity and Asthma Risk", THE NEW ENGLAND JOURNAL OF MEDICINE, - NEJM -, vol. 375, no. 19, 10 November 2016 (2016-11-10), US, pages 1897 - 1899, XP055601406, ISSN: 0028-4793, DOI: 10.1056/NEJMc1611699
- S ROY: "Bacterial DNA in house and farm barn dust", JOURNAL OF ALLERGY AND CLINICAL IMMUNOLOGY, vol. 112, no. 3, 1 September 2003 (2003-09-01), AMSTERDAM, NL, pages 571 - 578, XP055601401, ISSN: 0091-6749, DOI: 10.1016/S0091-6749(03)01863-3

## Description

### FIELD OF THE INVENTION

The present invention relates to a method of preparing a barn dust extract, said barn dust extract, and a composition comprising said barn dust extract. The barn dust extract is useful in the prevention or treatment of a disease.

### BACKGROUND

Allergy has developed into a major health concern in Europe with over 80 million people affected by some form of allergic disease and around 30 million people suffering from asthma. About 50% of all children (in some areas even 70%) have a positive allergy test to 'normal' exposures such as house dust, animals, pollen and food. The human immune system has become less able to tolerate these natural exposures and instead reacts with immunoglobulin E (IgE) antibodies, therefore developing allergic sensitization. These overshooting immune responses result in allergic airway disease such as hay fever and allergic asthma. The prevalence of hay fever is 20% among school children and 30% among teenagers. About 10% of children suffer from asthma. Most allergic patients develop a chronic course spreading into adolescence and adult age which results in significant health threats and severe limitations of activities and quality of life.

A way to prevent the new onset of asthma, hay fever and allergic sensitization is yet to be found, however there is compelling and consistent evidence across more than 30 studies worldwide that children growing up in a farming environment rich in microbial exposures are better protected from developing asthma, allergic rhinitis and atopic sensitization.

In the ALEX study the differences between farm and nonfarm children were: 1.4% versus 11.8% asthma, 3.2% versus 16% hay fever, 12.4% versus 32.9% allergic sensitization. This protection is sustained into adult life (von Mutius E and Vercelli D. Farm living: Effects on childhood asthma and allergy. Nat. Rev. Immunol. 10: 861-868 (2010) PMID: 21060319).

The farm effect on asthma can be explained by the child's early life contact to farm animals, mostly cattle (Illi S, Depner M, Genuneit J, et al. Protection from childhood asthma and allergy in Alpine farm environments - the GABRIEL Advanced Studies. J Allergy Clin Immunol 2012;129:1470-7 e6; Loss GJ, Depner M, Hose AJ, et al. The Early Development of Wheeze. Environmental Determinants and Genetic Susceptibility at 17q21. Am J Respir Crit Care Med 2016;193:889-97). These findings suggest that exposures encountered in animal sheds, in particular cattle stables, play a major role. Importantly, the epidemiological findings have been translated into experimental studies in mice thereby adding biological validity to these observations. In two independent studies, dust from a Bavarian cow shed (Schuijs MJ, Willart MA, Vergote K, et al. Farm dust and endotoxin protect against allergy through A20 induction in lung epithelial cells. Science 2015;349:1106-10) and aqueous extract of cow shed dust collected in the ALEX study (Peters M, Kauth M, Scherner O, et al. Arabinogalactan isolated from cowshed dust extract protects mice from allergic airway inflammation and sensitization. J Allergy Clin Immunol 2010;126:648-56 e1-4) were administered in aerosolized form (Peters M, Kauth M, Scherner O, et al. Arabinogalactan isolated from cowshed dust extract protects mice from allergic airway inflammation and sensitization. J Allergy Clin Immunol 2010;126:648-56 e1-4) or intranasally (Schuijs MJ, Willart MA, Vergote K, et al. Farm dust and endotoxin protect against allergy through A20 induction in lung epithelial cells. Science 2015;349:1106-10) in ovalbumin (OVA)-induced allergic asthma model (Peters M, Kauth M, Scherner O, et al. Arabinogalactan isolated from cowshed dust extract protects mice from allergic airway inflammation and sensitization. J Allergy Clin Immunol 2010;126:648-56 e1-4) or house dust mite (Schuijs MJ, Willart MA, Vergote K, et al. Farm dust and endotoxin protect against allergy through A20 induction in lung epithelial cells. Science 2015;349:1106-10) models of allergic asthma. In all studies this prophylactic treatment resulted in dramatically reduced airway hyperresponsiveness, suppressed eosinophilia and reduced levels of IgE, all hallmarks of allergic asthma. US 2008/112983 A1 discloses a method for producing an extract used for the treatment of allergic diseases. According to said method, no heat is delivered during the entire production process. Also disclosed are an extract that is produced according to the method as well as the use of said extract for producing a medicament. Peters M et al., Journal of Allergy and Clinical Immunology, Elsevier, Amsterdam, NL, vol. 126, no. 3, 1 September 2010, pages 648-656.e4, discloses that arabinogalactan isolated from cowshed dust extract protects mice from allergic airway inflammation and sensitization. M. J. Schuijs et al., Science, vol. 349, no. 6252, 4 September 2015, pages 1106-1110, discloses that farm dust and endotoxin protect against allergy through A20 induction in lung epithelial cells. Ober Carole et al., Current Opinion in Immunology, vol. 48 , pages 51-60, relates to immune development and environment and lessons from Amish and Hutterite children in this context.

Allergy therapies must often be administered over years and decades, and the application of newly developed biologicals results in exploding costs, therefore there is a need of developing preventive cures as they represent the only true long-term solution. Therefore, the technical problem underlying the present invention is the provision of further therapies for allergy development, in particular preventive therapies.

### SUMMARY OF THE INVENTION

The present invention is defined by the appended claims and it relates to a method of preparing a barn dust extract, wherein the barn dust is from a cow barn, comprising: (d) providing a mixture comprising a barn dust and a liquid; and (e) isolating a fraction of the mixture, wherein the barn dust extract comprised in the fraction consists essentially of molecules having a molecular weight of at at least of 10 kDa.

The present invention also relates to a barn dust extract obtainable by a method described herein.

The present invention also relates to a composition comprising the barn dust extract of the invention, in particular a pharmaceutical composition.

The present invention also relates to a barn dust extract of the invention or a composition of the invention for use in the prevention or treatment of an allergic disease selected from the group consisting of asthma and hay fever.

### BRIEF DESCRIPTION OF THE DRAWINGS

**FIGURE 1**

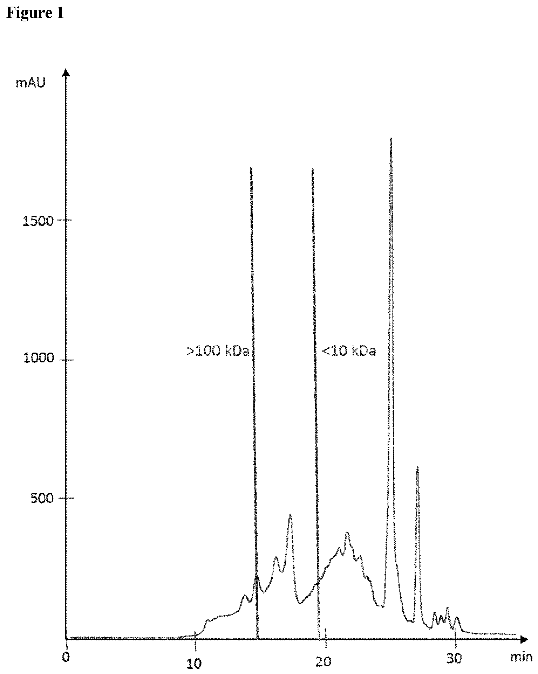

   Figure 1 shows a HPLC chromatogram of the stable dust total extract that has been not lyophilized and not autoclaved.
   Equipment and parameters:
   Stable dust: Hechfellner Hof 2017.
   System: Agilent Series 1100.
      Diode array detector: wavelength 210 nm.
      Columns: Agilent Advance Bio SEC 130 Å + Agilent Advance Bio SEC 300 Å (each: length 30 cm, diameter: 4.6 mm, particle size 2.7 µm).
      Column oven temperature: 30 °C.
      Mobile phase: 0.15 M NaCl with bidest. H₂O.
      Flow rate 0.3 mL/min.
      Injection volume: 10 µL.
**FIGURE 2**

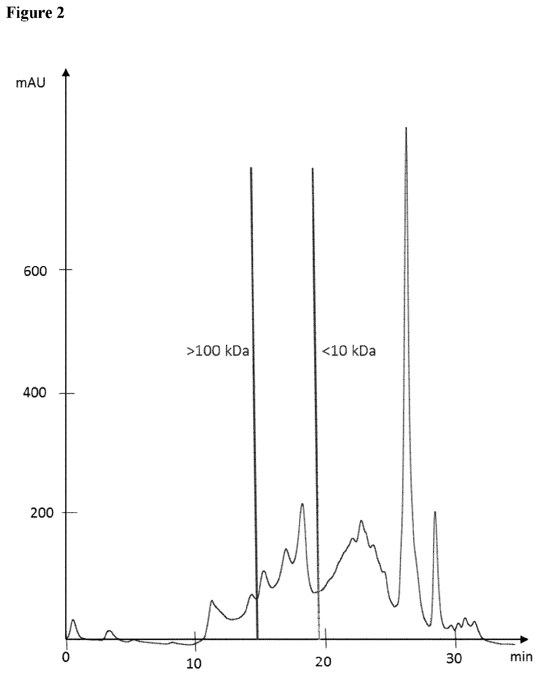

   Figure 2 shows a HPLC chromatogram of the stable dust total extract that has been lyophilized and not autoclaved.
   Equipment and parameters:
   Stable dust: Hechfellner Hof 2017.
   System: Agilent Series 1100.
      Diode array detector: wavelength 210 nm.
      Columns: Agilent Advance Bio SEC 130 Å + Agilent Advance Bio SEC 300 Å (each: length 30 cm, diameter: 4.6 mm, particle size 2.7 µm).
      Column oven temperature: 30 °C.
      Mobile phase: 0.15 M NaCl with bidest. H₂O.
      Flow rate 0.3 mL/min.
      Injection volume: 10 µL (12.3 mg/mL lyophilized dust in water).
**FIGURE 3**

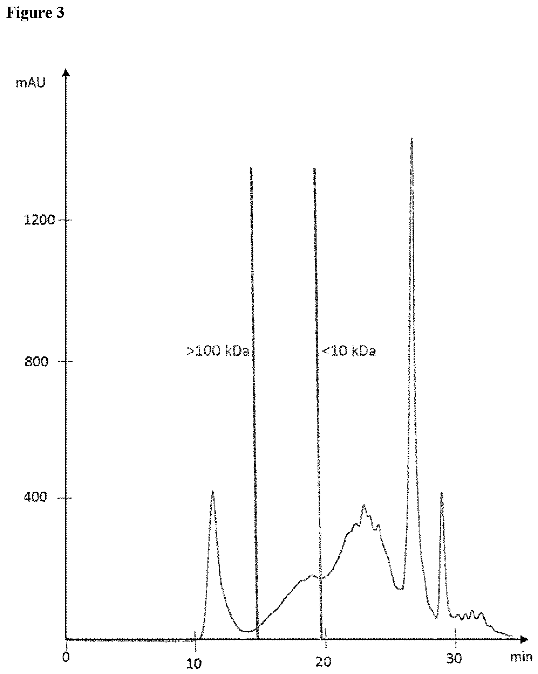

   Figure 3 shows a chromatogram of the stable dust Total extract that has been lyophilized and autoclaved.
   Equipment and parameters:
   Stable dust: Hechfellner Hof 2017.
   System: Agilent Series 1100.
      Diode array detector: wavelength 210 nm.
      Columns: Agilent Advance Bio SEC 130 Å + Agilent Advance Bio SEC 300 Å (each: length 30 cm, diameter: 4.6 mm, particle size 2.7 µm).
      Column oven temperature: 30 °C.
      Mobile phase: 0.15 M NaCl with bidest. H₂O.
      Flow rate 0.3 mL/min.
      Injection volume: 10 µL (9.9 mg/mL lyophilized dust in water).
**FIGURE 4**

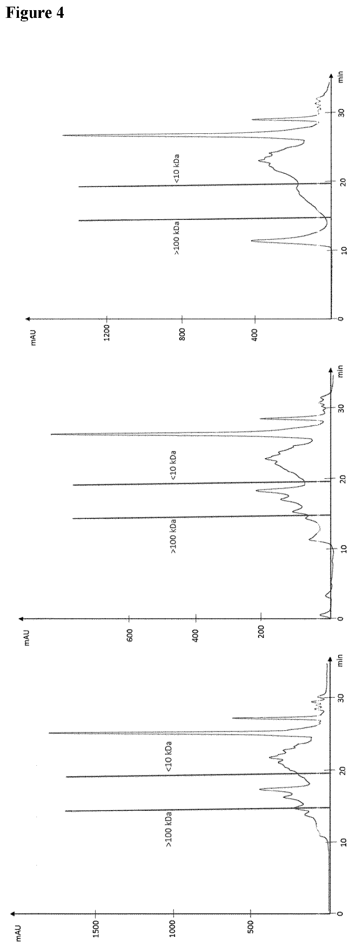

   Figure 4 provides an overview allowing comparison of the chromatograms depicted in Figures 1, 2 and 3.
**FIGURE 5**

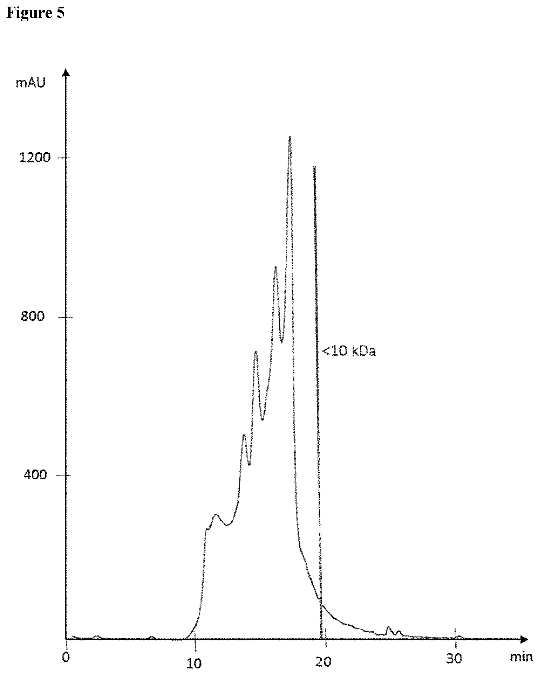

   Figure 5 shows a chromatogram of the > 10 kDa fraction of a stable dust extract that has not been lyophilized and not autoclaved.
   Equipment and parameters:
   Stable dust: Hechfellner Hof 2017.
   System: Agilent Series 1100.
      Diode array detector: wavelength 210 nm.
      Columns: Agilent Advance Bio SEC 130 Å + Agilent Advance Bio SEC 300 Å (each: length 30 cm, diameter: 4.6 mm, particle size 2.7 µm).
      Column oven temperature: 30 °C.
      Mobile phase: 0.15 M NaCl with bidest. H₂O.
      Flow rate 0.3 mL/min.
      Injection volume: 10 µL.
**FIGURE 6**

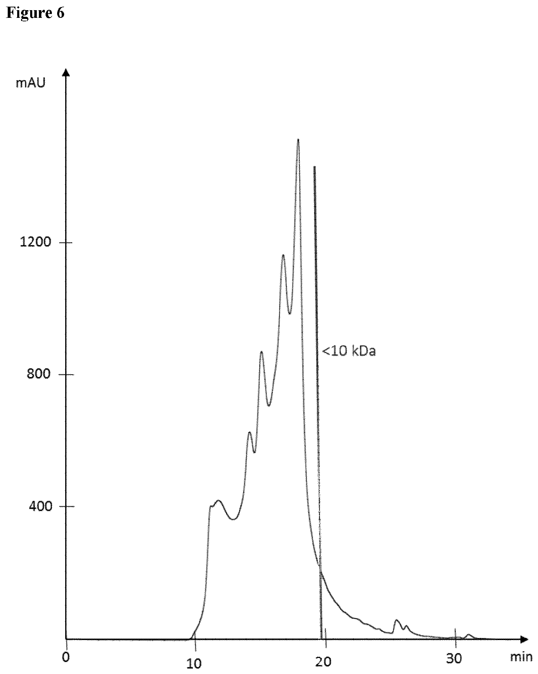

   Figure 6 shows a chromatogram of the > 10 kDa fraction of a stable dust extract that has been lyophilized and not autoclaved.
   Equipment and parameters:
   Stable dust: Hechfellner Hof 2017.
   System: Agilent Series 1100.
      Diode array detector: wavelength 210 nm.
      Columns: Agilent Advance Bio SEC 130 Å + Agilent Advance Bio SEC 300 Å (each: length 30 cm, diameter: 4.6 mm, particle size 2.7 µm).
      Column oven temperature: 30 °C.
      Mobile phase: 0.15 M NaCl with bidest. H₂O.
      Flow rate 0.3 mL/min.
      Injection volume: 10 µL (13.0 mg/mL lyophilized dust in water).
**FIGURE 7**

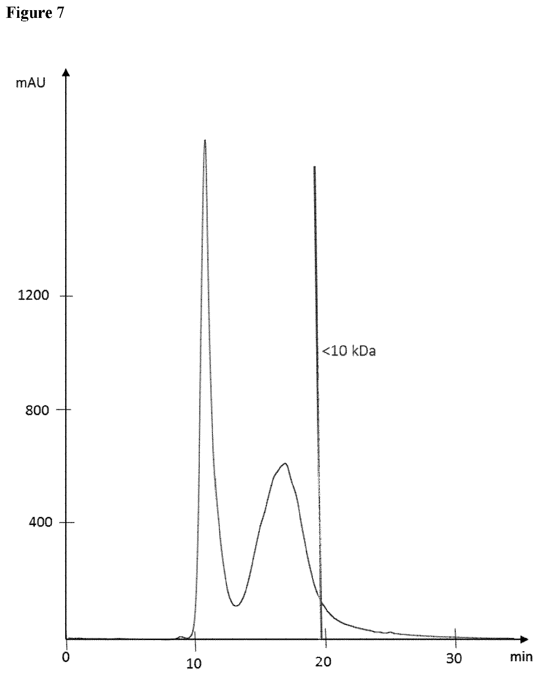

   Figure 7 shows a chromatogram of the > 10 kDa fraction of a stable dust extract that has been lyophilized and autoclaved. Equipment and parameters:
   Stable dust: Hechfellner Hof 2017.
   System: Agilent Series 1100.
      Diode array detector: wavelength 210 nm.
      Columns: Agilent Advance Bio SEC 130 Å + Agilent Advance Bio SEC 300 Å (each: length 30 cm, diameter: 4.6 mm, particle size 2.7 µm).
      Column oven temperature: 30 °C.
      Mobile phase: 0.15 M NaCl with bidest. H₂O.
      Flow rate 0.3 mL/min.
      Injection volume: 10 µL (8.4 mg/mL lyophilized dust in water).
**FIGURE 8**

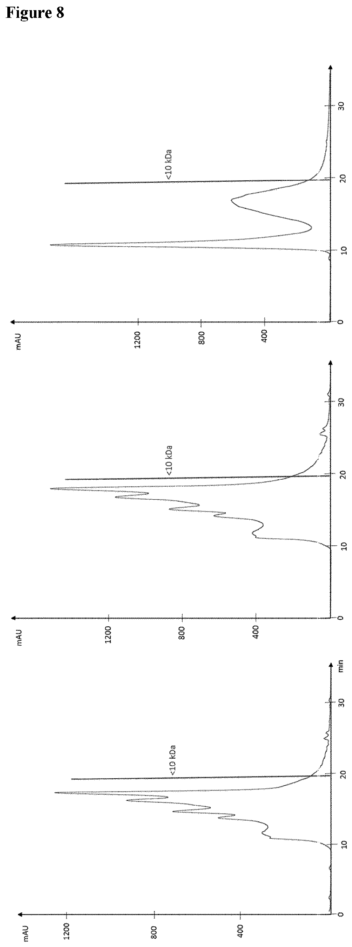

   Figure 8 provides an overview allowing comparison of the chromatograms depicted in Figures 5, 6 and 7.
**FIGURE 9**

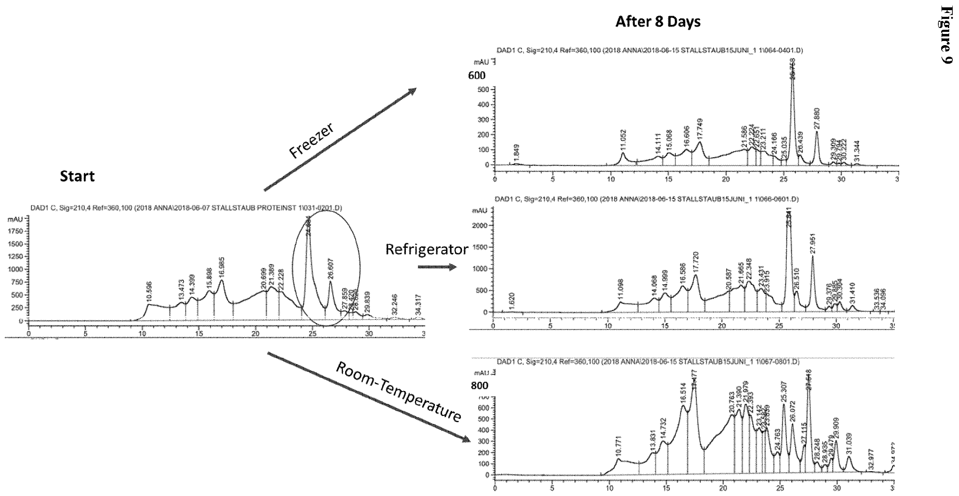

   Figure 9 illustrates the change in liquid extract (not autoclaved) measured after 8 days of storage under different conditions, namely storage at room temperature, refrigerated (storage at 4 °C) and freezed (storage at -20 °C)
**FIGURE 10**

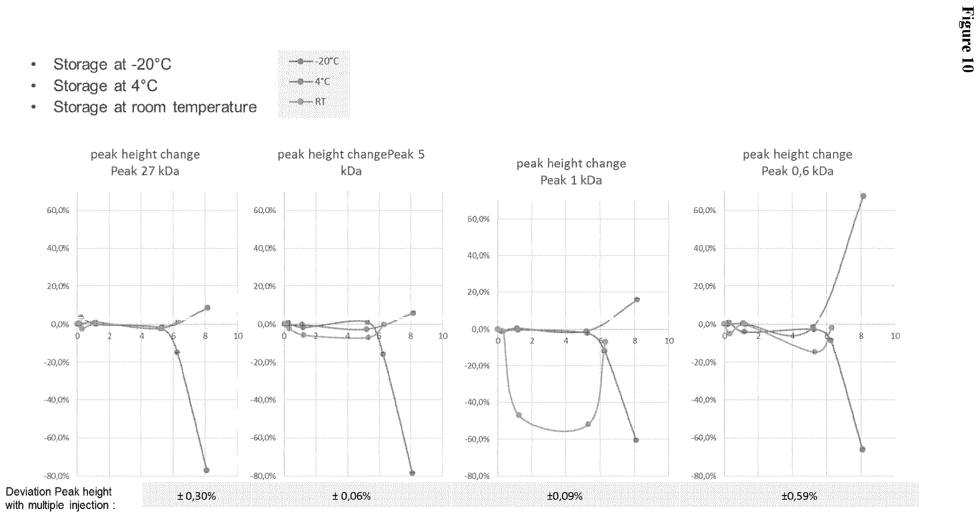

   Figure 10 illustrates the change in peak height for the peaks of respectively 27 kDA, 5 kDA, 1 kDa, and 0.6 kDa, measured after 8 days of storage under different conditions, namely storage at room temperature, storage at 4 °C and storage at -20 °C.
**FIGURE 11**

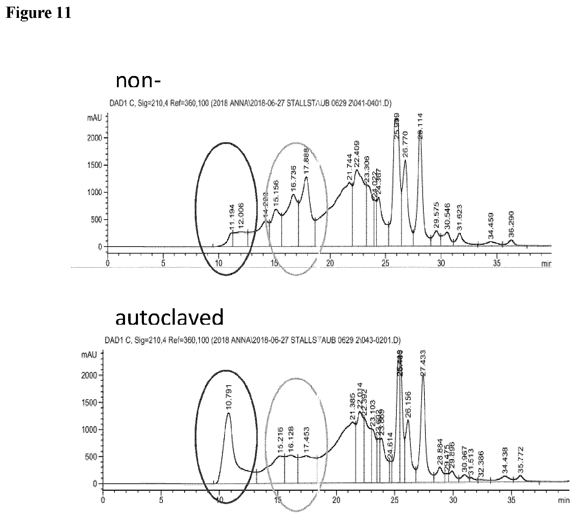

   Figure 11 illustrates the difference between non-autoclaved and autoclaved liquid extracts obtained from barn dust from Hechfellner Hof.
**FIGURE 12**

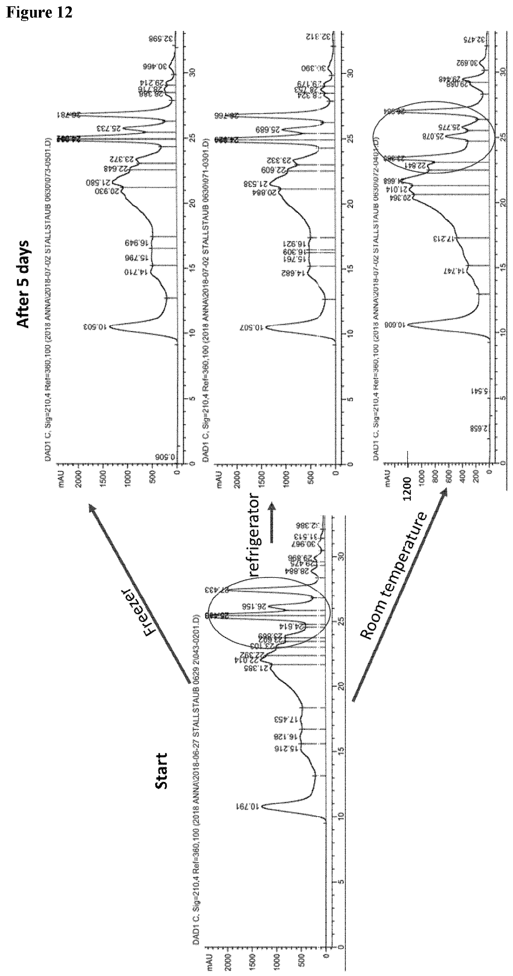

   Figure 12 illustrates the change in liquid extract (autoclaved) measured after 5 days of storage under different conditions, namely storage at room temperature, storage at 4 °C and storage at -20 °C.
**FIGURE 13**

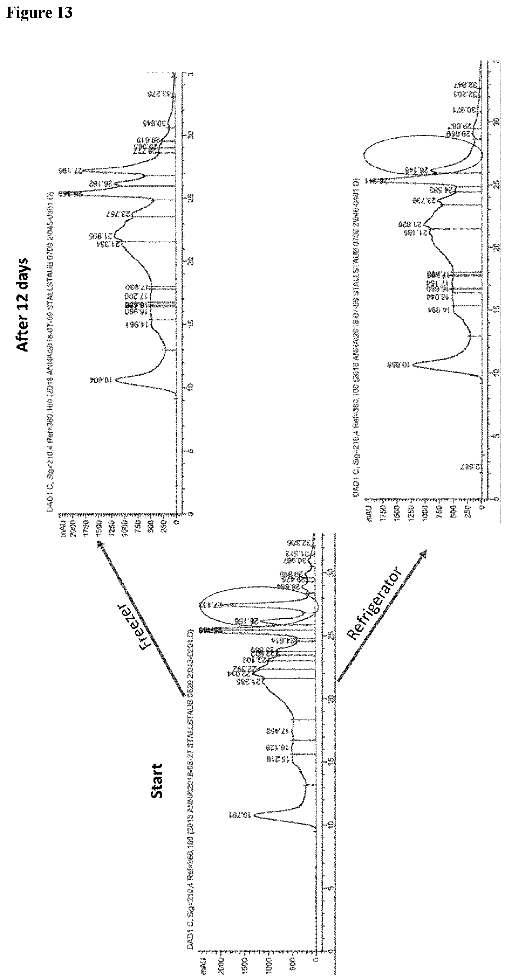

   Figure 13 illustrates the change in liquid extract (autoclaved) measured after 12 days of storage under different conditions, namely storage at room temperature, storage at 4 °C and storage at -20 °C.
**FIGURE 14**

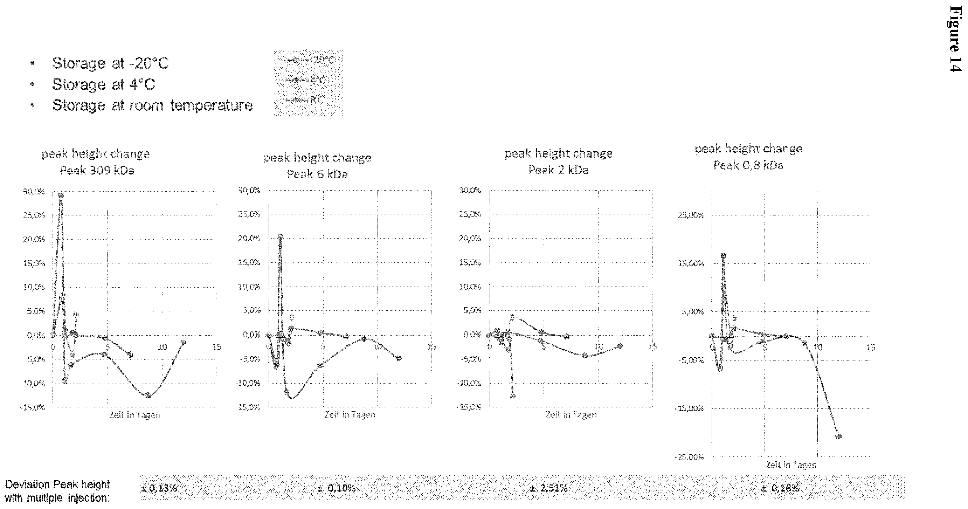

   Figure 14 illustrates the change in peak height for the peaks of respectively 309 kDa, 6 kDa, 2 kDa, and 0.8 kDa, measured after 8 days of storage under different conditions, namely storage at room temperature, storage at 4° and storage at -20°.
**FIGURE 15**

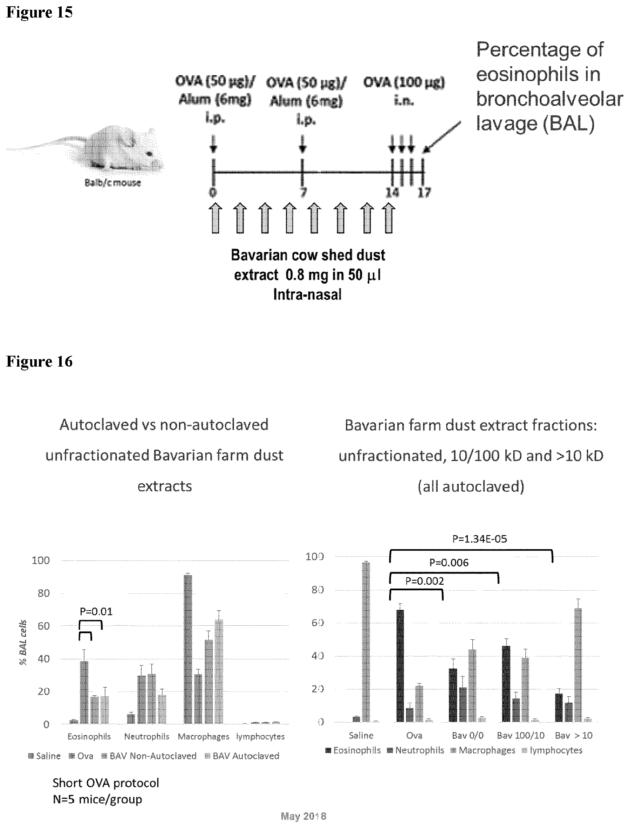

   Figure 15 illustrates the experimental setup used to measure eosinophil recruitment to the lung in mice after intra-nasal administration of fractionated or unfractionated stable dust extracts. Dust extracts (0.8 mg dry weight in 50 µl/treatment) are instilled intra-nasally every 2-3 days for a total of 8 times beginning at day 0 into 7-week old Balb/c mice that are sensitized intra-peritoneally with 50 µg of OVA-Alum (6 mg) at day 0 and 7, and challenged intra-nasally with 100 µg OVA at day 14, 15, and 16. Broncho-alveolar lavage (BAL) is performed at day 17 by delivering cold 1% BSA in PBS (2 mL) into the airway via a tracheal cannula and gently aspirating the fluid. Cells are counted using a Countess II FL automated cell counter (Thermo Fisher Scientific) and differentials are determined by an operator blinded to mouse ID/grouping after staining with Hema 3 (Fischer) (at least 400 cells/slide). Statistical significance for BAL cellularity measurements is determined by an unpaired two tailed Student's t-test. P-values <0.05 are considered significant. Each treatment group includes a minimum of 5 mice
**FIGURE 16**
   Left panel of figure 16 illustrates eosinophils count after treating mice with a saline solution (negative control), an allergen (OVA, positive control), unfractionated, autoclaved stable dust extract (BAV Autoclaved), and unfractionated, non-autoclaved stable dust extract (BAV Non-Autoclaved).
   In the right panel are reported eosinophils counts after treating mice with a saline solution (negative control), an allergen (OVA, positive control), unfractionated, autoclaved barn dust extract (Bav 0/0), fractions between 10 kDa and 100 kDa of autoclaved stable dust extract (Bav 100/10), and fractions bigger than 10 kDa of autoclaved stable dust extract (Bav >10).
**FIGURE 17**

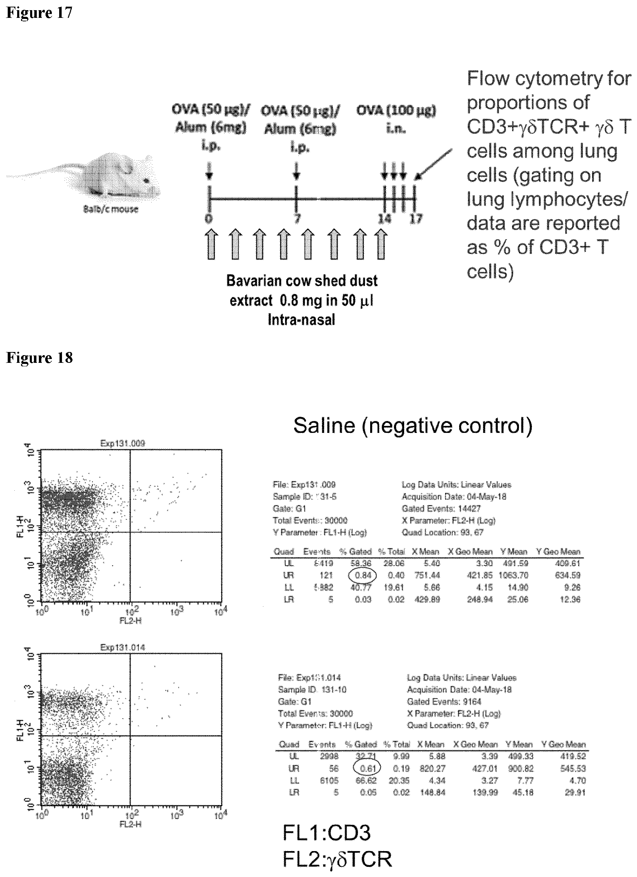

   Figure 17 illustrates the experimental setup used to measure proportions of γδ T cells in the lungs of mice following intra-nasal administration of a saline solution (negative control), an allergen (OVA, positive control), unfractionated, autoclaved barn dust extract, fractions between 10 kDa and 100 kDa of autoclaved stable dust extract, and fractions bigger than 10 kDa of autoclaved stable dust extract.
   Mice are euthanized by lethal dose of anesthetic. Lungs are perfused and removed. To prepare single-cell suspension the lungs are minced with scissors and digested with 0.26 Wunsch U/ml of Liberase TM (Roche) and 4 U/ml of DNAse I (Sigma) at 37°C for 1 h with shaking (60-70 rpm). The resulting suspension is passed through 20-22G needle and through the 70 µm cell strainer. After washing with complete medium, lung cells are resuspended in PBS/0.1% NaN3/1.0% BSA at 5x10⁶ cells/ml. Cells are treated with Fc-Block for 10 min on ice and stained with fluorescently labeled antibodies to cell surface antigens for 30 min on ice in the dark. Cells are then washed and resuspended in PBS/0.1% NaN3/1.0% BSA for flow cytometry.
   The following antibodies to cell surface antigens are used: PE or BV510 conjugated hamster anti-mouse γδ T-Cell Receptor (GL3), FITC-hamster anti-mouse CD3ε (145-2C11), PE-Cy7 or PE-Cy5-rat anti-mouse CD4 (RM4-5), APC-R700-rat anti-mouse CD8α (53-6.7). All from BD Pharmingen. Flow cytometry is performed on a FACSCalibur or LSRII (BD Sciences) flow cytometers. Data is collected on 10000-20000 events. Data analysis is performed using CellQuest (BD) or FlowJo (FlowJo) software. Proportions of γδ T cells are reported as % of CD3+ T cells gating on lung lymphocytes.
**FIGURE 18**
   Figure 18 illustrates the results of flow cytometry analysis to determine the proportion of CD3⁺γδ⁺ cells found in the lungs after treating mice with a saline solution (negative control). CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.
**FIGURE 19**

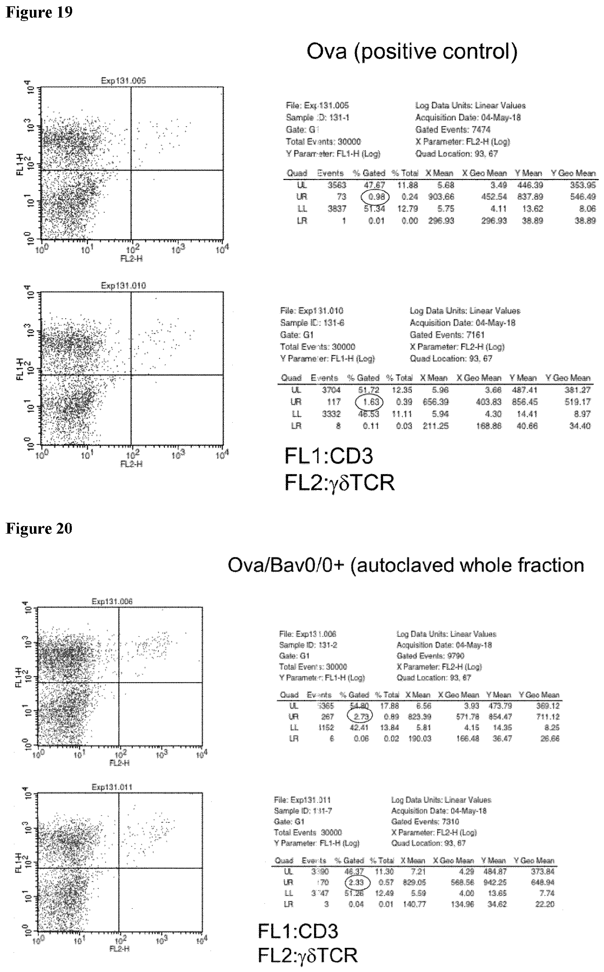

   Figure 19 illustrates the results of flow cytometry analysis to determine the proportion of CD3⁺γδ⁺ cells found in the lungs after treating mice with an allergen (OVA). CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse
**FIGURE 20**
   Figure 20 illustrates the results of flow cytometry analysis to determine the proportion of CD3⁺γδ⁺ cells found in the lungs after treating mice with allergen (OVA) + unfractionated, autoclaved barn dust extract. CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse
**FIGURE 21**

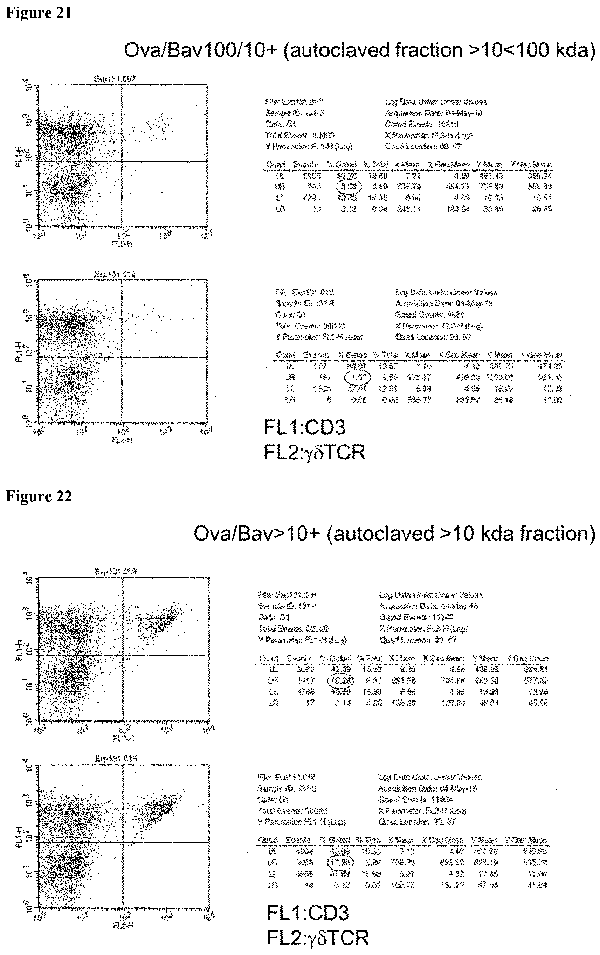

   Figure 21 illustrates the results of flow cytometry analysis to determine the proportion of CD3⁺γδ⁺ cells found in the lungs after treating mice with allergen (OVA) + fractions of autoclaved stable dust extract between 10 kDa and 100 kDa. CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.
**FIGURE 22**
   Figure 22 illustrates the results of flow cytometry analysis to determine the proportion of CD3⁺γδ⁺ cells found in the lungs after treating mice with allergen (OVA) + fractions of autoclaved stable dust extract > 10 kDa. CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.

### DETAILED DESCRIPTION

The present invention is based on the surprising finding that certain processing step(s) may improve the quality and/or anti-allergenic potential of a barn dust (extract). The inventors of the present application have surprisingly found that certain fractions of barn dust extracts obtained after fractionation according to molecular weight of barn dust components exhibit an improved anti-allergenic potential as compared to the whole extract.

According to the surprising finding the present invention relates to a method of preparing a barn dust extract, wherein the barn dust is from a cow barn, comprising: (d) providing a mixture comprising a barn dust and a liquid; (e) isolating a fraction of the mixture, wherein the barn dust extract comprised in the fraction consists essentially of molecules having a molecular weight of at least 10 kDa.

The term "barn dust" as used herein, relates to dust that can be, is, or has been collected from a barn. Without wishing to be bound by theory it is believed that barn dust comprises immunostimulatory substances derived from microorganisms, animals, plants, fungi, viruses and/or protozoa that are protective against allergies, asthma and/or other diseases disclosed herein. In preferred embodiments, the barn dust is from a farm. Generally, the origin of a barn dust is not limited to certain types of barns and can be any type of barn, including barns for any type of livestock such as cows, pigs, chicken, sheep, horse, or others. In the context of the methods of the claimed invention, the barn dust is from a cow barn. Also, the geographic location of the barn is believed to be not essential for the invention. Exemplary non-limiting geographic locations for a barn are Europe, including the member states of the European Union, such as Germany, France, Austria, Switzerland, Czech Republic, Poland, the Netherlands, Belgium, Luxemburg, Spain, Portugal, Italy, etc., the barn may for example be located in Bavaria, Germany. As a non-limiting example, barn dust may be obtained from Hechfellner Hof, Mettenheim, Bavaria, Germany. Barn dust can be collected by any suitable method known to the person skilled in the art optionally by applying any type of collection system that is suitable for collecting barn dust. Barn dust can for example be collected by sweeping, vacuuming, or swiping. Barn dust can also be collected by filtration of barn air, for example by using the membrane filter or a granular material that is capable of adsorbing barn dust. Barn dust can also be collected using an impinge or an impactor, such as a cascade impactor.

After collection, the dust may be homogenized. A suitable type of technique is any technique which leads to a uniform homogenization of the dust, in particular one which removes agglomerations and lumps of the dust. Suitable for these purposes are methods such as rubbing, smashing or crushing, stirring or introducing into a blender, without being limited thereto. Digesting the dust may also be part of the process according to the invention. Digesting of the constituents present in the dust, such as cells, microorganisms, in particular their spores and the like can be effected, for example by grinding, squashing and similar methods. A sieving step for removing (large) particles, such as particles having a size of about 100 µm, about 90 µm, about 80 µm, about 70 µm, about 60 µm, about 50 µm, about 40 µm, about 30 µm, about 20 µm, or about 10 µm may be applied after homogenization.

The term "barn dust extract" as used herein preferably refers to a composition that is obtainable by the methods disclosed herein and may refer to both, a solution or suspension, or a dry composition.

For the methods of the present invention the barn dust is in a mixture with a liquid. The liquid may be water, an aqueous solution, or a water miscible solvent, or a combination thereof. It can be pure water, but it can also be a saline solution. A saline solution may contain sodium salts or similar monovalent or divalent salts, such as sodium sulfate, potassium chloride, magnesium chloride, or calcium chloride. A preferred saline solution is a solution of sodium chloride, preferably a physiologic aqueous solution of sodium chloride, such as normal saline. The term "normal saline" as used herein preferably refers to a mixture of sodium chloride and water at a concentration of about 9 g/L. The aqueous solution may also comprise a buffer, such as phosphate buffered saline. The water-miscible solvent may for example be an alcohol such as ethanol. The water-miscible solvent may also be a mixture of water-miscible solvents. The liquid may also be a mixture of water and/or an aqueous solution and one or more water-miscible solvent(s).

The term "fraction" as used herein preferably refers to fractions that can be obtained by fractioning a mixture according to the molecular weight and/or size of the molecules comprising the mixture.

The term "isolating" or "isolation" as used herein preferably refers to enriching a portion that is to be isolated as compared to a portion that is not to be isolated by mass, or depleting a portion that is not to be isolated as compared to a portion that is to be isolated by mass. Accordingly, step (e) of the methods of the present invention may comprise enriching a fraction wherein the barn dust extract comprised therein consists essentially of molecules having a molecular weight of at least 10 kDa by factor of at least about 5, preferably at least about 10, preferably at least about 20, preferably at least about 100. Step (e) of the methods of the present invention may also comprises depleting a fraction wherein the barn dust extract comprised therein consists essentially of molecules having a molecular weight of at most 10 kDa by factor of at least about 5, preferably at least about 10, preferably at least about 20, preferably at least about 100.

The term "consists essentially of" means that further components, such as impurities, can be present but that the further components do not markedly affect the essential characteristics of the components that the mixture, fraction, compound etc. essentially consists of. Such further component(s) may amount to up to about 10%, up to about 9%, up to about 8%, up to about 7%, up to about 6%, up to about 5%, up to about 4%, up to about 3%, up to about 2%, up to about 1%, up to about 0.5%, up to about 0.2%, or up to about 0.1% by weight of component(s) that the mixture, fraction, compound etc. essentially consists of.

The inventors of the present application have also surprisingly found that isolating a fraction, in which the barn dust comprised therein consists essentially of molecules having a molecular weight of at least about 5 kDa, preferably at least about 10 kDa, will yield a highly standardized product that is essentially independent from the origin of the starting material.

The method of the present invention may further comprise the step of subjecting a mixture comprising a barn dust and liquid to heat treatment. The heat treatment may be at a temperature of at least about 110 °C and/or a pressure of at least about 1.5 bar for at least about 3, 4, 5, 6, 7, 8, 9, or 10 minutes. The heat treatment may comprise subjecting the mixture to a moist heat, such as a saturated steam. The heat treatment may comprise autoclaving the mixture. Autoclaving may be performed using steam heated to about 121-134 °C, for at least about 3 minutes, such as at least about 5 minutes, at least about 8 minutes, at least about 10 minutes, at least about 15 minutes or at least about 20 minutes. Such a heat treatment may be performed for up to about 2 hours, up to about 1.5 hours, up to about 1 hour, up to about 45 min or up to about 30 min. A preferred heat treatment may be subjecting the mixture to moist heat at 121 °C (2.1 bar) for at least about 15 minutes (such as about 20 minutes), at 134°C (3 bar) for at least about 3 minutes (such as about 5 minutes), or at 134 °C (3 bar) for at least about 18 minutes. The latter conditions may further inactivate prions. Heat treatment can also be performed using dry heat, for example at about 160 °C to about 200 °C for up to about two hours. Optimal duration for dry heat treatment may vary depending of the temperature. Typical conditions for dry heat treatment are for example about 2 h at about 160 °C, or about 1 h at about 170 °C, or about 6 to 12 minutes at 190 °C.

The inventors of the present application have surprisingly found that heat treatment alters the molecular weight distribution of the molecules comprised in the barn dust extract. However, it has surprisingly been found that this altered molecular weight distribution does not essentially affect the therapeutic potency of the barn dust extract. However, heat treatment is believed to result in a sterile product that meets safety requirements for pharmaceuticals and also improves stability of the product. Alternatively or additionally, the barn dust extract may undergo sterile filtration. A sterile filtration step may be conducted before step (e). A sterile filtration step may also be conducted before step (g).

The methods of the present invention may further comprise step (g), drying the fraction obtained in step (e). "Drying" as used herein preferably refers to complete or partial removal of solvents, in particular water. The drying step can be performed by any means or methods known to the person skilled in the art. Non-limiting examples include lyophilization, spray drying, sun drying, or air drying. Preferred methods are lyophilization or spray drying. A dry barn dust extract is believed to be more stable than a liquid extract and is thus particularly suited for long-term storage.

The methods of the present invention may comprise the step of (a) collecting barn dust. This collecting step can be performed by any method described here in. After collection of the barn dust, the barn dust may be mixed with a liquid described herein.

After mixing the dust with the liquid, this suspension may be either left to stand or stirred, so that the soluble substances, or the substances which can be removed from the dusts by the liquid, may enter into the liquid phase.

Thereafter, insoluble substances may be subsequently removed from the mixture. Removal can for example be conducted by filtration or a sedimentation step. The removal step is not limiting for the process according to the invention; any removal method which is known to the skilled worker may be employed. Also, leaving the suspension to stand so that sedimentation of the solid constituents can be accomplished by the earth's gravity may be considered as being for the purposes of the invention. A preferred way of removing the solid constituents is centrifuging, whereafter the supernatant is removed from the sediment.

Subsequently, the methods of the present invention may comprise step (c) depleting particles having a size of at least about 0.2 µm, which may comprise one or more filtration steps. Particles having a size of at least about 0.2 µm may be depleted by factor of at least about 10, preferably at least about 100, preferably at least about 1000.

The inventors of the present application have further surprisingly found that barn dust extract fractions that consists essentially of molecules having molecular weight of at least about 5 kDa, preferably at least about 10 kDa with no defined upper limit for the molecular weight have a higher therapeutic potency than fractions that consist essentially of molecules having molecular weight about 5 kDa to about 100 kDa or about 10 kDa to about 100 kDa. The maximum molecular weight or size of the molecules comprised in the barn dust extract may thus only be limited by the solubility of the molecules in water or the cut-off value of a filtration step in the preparation of the barn dust extract. Such a cut-off value may e.g. about 0.2 µm. Accordingly, the methods of the present invention preferably do not comprise the step of depleting molecules having a molecular weight of at least about 100 kDa from the barn dust extract. Accordingly the barn dust extract that consists essentially of molecules having a molecular weight of at least about 5 kDa, preferably at least about 10 kDa, preferably also comprises molecules having a molecular weight of at least about 100 kDa.

The methods of preparing a barn dust extract of the present invention may therefore comprise steps of:
(a) (optionally) collecting barn dust;
(b) (optionally) mixing barn dust with a liquid;
(c) (optionally) depleting particles having a size of at least about 0.2 µm;
(d) providing a mixture comprising a barn dust and a liquid;
(e) isolating a fraction of the mixture, wherein the barn dust extract comprised in the fraction consists essentially of molecules having a molecular weight of at least 10 kDa; and
(f) (optionally) subjecting the mixture to heat treatment;
(g) (optionally) drying the fraction obtained in step (f).

The present invention further relates to a barn dust extract. The barn dust extract is obtainable by a method of the invention.

A barn dust extract of the invention may consist to at least about 90% by dry matter of molecules having a molecular weight of at least about 5 kDa, preferably at least about 10 kDa. Such a barn dust extract has a distinct molecular weight distribution of the molecules comprised in the extract. A size exclusion chromatogram of the barn dust extract has in the fraction having a molecular weight of at least about 10 kDa two characteristic peaks A and B, wherein peak A has its maximum in the range of 35 to 75 kDa (corresponding to a retention time of about 17 min, preferably as measured in an size exclusion chromatography assay as described herein); and (b) peak B has its maximum in the range of 300 to 700 kDa (corresponding to a retention time of about 11 min, preferably as measured in an size exclusion chromatography assay as described herein), as shown e.g. in Figure 7.

Size exclusion chromatography is preferably carried out using an Agilent Advance Bio SEC 130 Å (2.7 µm, 4.6 x 300 mm) and/or an Agilent Advance Bio SEC 300 Å (2.7 µm, 4.6 x 300 mm) column and a 0.15 M NaCl solution as mobile phase, and wherein compounds are detected by an optical detector at a wavelength of 210 nm. Further conditions that are believed to be non-essential may be an injection volume (sample) of 10 µL, a column temperature of 30 °C, and/or a flow rate of 0.3 mL/min.

The barn dust extract of the invention may have undergone heat treatment as defined in the methods of the invention.

The barn dust extract of the invention may consist essentially of molecules having a molecular weight of at least 10 kDa. Accordingly, the barn dust extract may consist consists to at least about 75%, preferably 80%, preferably 85%, preferably 90%, preferably 95%, preferably 98%, preferably 99%, preferably 99.5%, preferably 99.9% by dry matter of molecules having a molecular weight of at least about 5 kDa. The barn dust extract preferably consists to at least about 75%, preferably 80%, preferably 85%, preferably 90%, preferably 95%, preferably 98%, preferably 99%, preferably 99.5%, preferably 99.9% by dry matter of molecules having a molecular weight of at least about 10 kDa.

The barn dust extract disclosed herein preferably comprises one or more bacterial antigens. It can further comprise fungal, viral or protozoal antigens. A barn dust or barn dust extract disclosed herein may comprises a fragment of a bacterium selected from the group consisting of *Staphylococcus sciuri, Jeotgalicoccus* spp., preferably *J*. *halotolerans, J. pinnipedialis, J. psychrophilus, Salinicoccus alkaliphilus, Salinicoccus halodurans, Salinicoccus kunmingensis, Salinicoccus roseus, Macrococcusbrunensis, Duganella* spp., preferably *D. violaceinigra, D. zoogloeoides, Moraxella boevrei, Moraxella canis, Moraxella caprae, Moraxella cuniculi, Moraxella lincolnii, Corynebacterium variabile, Corynebacterium freiburgense,* and *Zooglea* spp., preferably *Z*. *caeni, Z. ramigera, Z. resiniphila,* or a mixture thereof. Alternatively or additionally, a barn dust or barn dust extract disclosed herein may comprise a fragment of a bacterium selected from the group consisting of *Lactobacillus* spp., preferably *Lactobacillus curvatus, Lactobacillus sakei,* and *Lactobacillus iners, Delftia* spp., preferably *D. tsuruhatensis, Brevibacterium* spp., preferably *B. iodinum, B. linens, Rhizobium* spp., preferably *R. gallicum, Psychromonas* spp., *Alteromonas* spp., *Lactococcus lactis,* and *Acinetobacter* spp., preferably *Acinetobacter lwoffii* or mixtures thereof

The present invention also relates to a composition comprising a barn dust extract of the present invention.

Such a composition may be a pharmaceutical composition comprises the barn dust extract as active ingredient and optionally, one or more pharmaceutically excipient(s). Accordingly, the use of a barn dust extract described herein, for the manufacture of a pharmaceutical composition or medicament is also envisaged herein.

The term "pharmaceutical composition" particularly refers to a composition suitable for administering to a human. However, compositions suitable for administration to non-human animals are generally also encompassed by the term.

The pharmaceutical composition and its components (i.e. active agents and optional excipients) are preferably pharmaceutically acceptable, i.e. capable of eliciting the desired therapeutic effect without causing any undesirable local or systemic effects in the recipient. Pharmaceutically acceptable compositions of the invention may for instance be sterile or non-sterile. Specifically, the term "pharmaceutically acceptable" may mean approved by a regulatory agency or other generally recognized pharmacopoeia for use in animals, and more particularly in humans.

The barn dust extract is preferably present in the pharmaceutical composition in a therapeutically effective amount. By "therapeutically effective amount" is meant an amount of the active agent that elicits the desired therapeutic or prophylactic effect. Therapeutic efficacy can be determined by standard procedures, e.g. in test animals, e.g., ED₅₀ (the dose therapeutically effective in 50% of the population).

The term "excipient" includes fillers, binders, disintegrants, coatings, sorbents, antiadherents, glidants, preservatives, antioxidants, flavoring, coloring, sweeting agents, solvents, co-solvents, buffering agents, chelating agents, viscosity imparting agents, surface active agents, diluents, humectants, carriers, diluents, preservatives, emulsifiers, stabilizers and tonicity modifiers. It is within the knowledge of the skilled person to select suitable excipients for preparing the desired pharmaceutical composition of the invention. Exemplary carriers for use in the pharmaceutical composition of the invention include saline, buffered saline, dextrose, and water. Typically, choice of suitable excipients will *inter alia* depend on the specific active agent used, the disease to be treated, and the desired formulation of the pharmaceutical composition.

The exact dosage of the barn dust extract described herein (or the pharmaceutical composition comprising the same), will be ascertainable by one skilled in the art using known techniques. Suitable dosages provide sufficient amounts of the barn dust extract and are preferably therapeutically effective.

The compositions of the invention can be formulated in various forms, e.g. in solid, liquid, gaseous or lyophilized form and may be, for instance, in the form of a solution, an aerosol, a suspension, a lyophilisate, a powder, a tablet, a dragee, a suppository, a pill, a capsule, granule, an ointment, a cream, transdermal patches, a gel, suspensions, emulsions, syrups, liquids, elixirs, extracts, tincture or fluid extracts or in a form which is particularly suitable for the desired method of administration.

A variety of routes are applicable for administration of the composition according to the present invention. Typically, the composition may be prepared for nasal, inhalative, oral, conjunctival, subcutaneous, intraarticular, intraperitoneal, rectal, or vaginal administration. The composition may be in a form of a food additive, a food ingredient, or a composition suitable to be distributed in indoor air.

A barn dust extract of the invention or a composition of the invention, in particular a pharmaceutical composition, may be for use in the prevention or treatment of a disease. The use preferably comprises administering the barn dust extract or the composition to a subject that is preferably in need thereof.

The term "treatment" in all its grammatical forms includes therapeutic or prophylactic treatment of a subject in need thereof. A "therapeutic or prophylactic treatment" comprises prophylactic treatments aimed at the complete prevention of clinical and/or pathological manifestations or therapeutic treatment aimed at amelioration or remission of clinical and/or pathological manifestations. The term "treatment" thus also includes the amelioration or prevention of diseases.

The disease may be selected from the group consisting of an allergic disease selected from the group consisting of asthma and hay fever.

It is envisioned by the invention that the "subject" may be an animal, preferably a vertebrate, preferably a mammal. Preferred subjects include human, mouse, rat, rabbit, hamster, pig, dog, cat, cattle, sheep, goat, camel, monkey, or ape. It is envisioned by the invention that a human subject is preferred over other species, wherein a baby, and infant, or a pregnant woman is most preferred.

Unless otherwise indicated, the term "at least" preceding a series of elements is to be understood to refer to every element in the series.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

**In** each instance herein any of the terms "comprising", "consisting essentially of" and "consisting of" may be replaced with either of the other two terms.

### EXAMPLES

### Example 1: Method of extract preparation from stable dust

Barn dust extracts may be prepared, fractionized, lyophilized and analyzed according to following method.
Extract preparation
   1. Crushing barn dust using a mixer and with addition of dry ice.
   2. Sieving of barn dust with two sieves (100 µm and 40 µm particle retention) (optional).
   3. Suspending 3 g barn dust in 30 mL Millipore water (100 mg barn dust/liter)
   4. Shaking suspension at 1000 rpm for two hours at room temperature.
   5. Centrifugation of Suspension for 5 minutes at 2500 × g at room temperature.
   6. Filtration of supernatant with vaccum pump and cellulose filter (particle retention 5-13 µm). Use of multiple filters.
   7. Second filtration with vacuum pump and cellulose filter (particle retention 2 µm). Use of multiple filters.
   8. Filtration with further filtration system (particle retention: 0.22 µm).
Fractionation
   9. Ultracentrifugation with 100 kDa membrane at 3000 × g at room temperature (optional, only for preparation of 100/10 kDa fractions).
   10. Ultracentrifugation with 10 kDa membrane at 3000 × g at room temperature.
Autoclaving
   11. Autoclaving (20 min at 121 °C, saturated steam).
Lyophilization
   12. Sterile filtration using syringe filters.
   13. Dispensing 3 mL sample per vial under sterile conditions.
   14. Lyophilization of samples
Analysis
   15. SEC analysis
   a. dissolving sample in Millipore water.
   b. measuring the sample (eluent: 0.15M NaCl, T 30 °C, flow: 0.3 mL/min, combination of 130 Å und 300 Å column, wavelength: 210 nm)

### Example 2: Effects of lyophilization and/or autoclaving on the chromatographic profile of a total stable dust extract and a stable dust extract comprising fractions >10 kDa.

Unless otherwise stated, barn dust extracts were prepared as described in Example 1.

Figure 1 shows a HPLC chromatogram of the stable dust total extract that has been not lyophilized and not autoclaved.
Equipment and parameters:
Stable dust: Hechfellner Hof 2017.
System: Agilent Series 1100.
   Diode array detector: wavelength 210 nm.
   Columns: Agilent Advance Bio SEC 130 Å + Agilent Advance Bio SEC 300 Å (each: length 30 cm, diameter 4.6 mm, particle size 2.7 µm).
   Column oven temperature: 30 °C.
   Mobile phase: 0.15 M NaCl with bidest. H₂O.
   Flow rate 0.3 mL/min.
   Injection volume: 10 µL.

Figure 2 shows a HPLC chromatogram of the stable dust total extract that has been lyophilized and not autoclaved.
Equipment and parameters:
Stable dust: Hechfellner Hof 2017.
System: Agilent Series 1100.
   Diode array detector: wavelength 210 nm.
   Columns: Agilent Advance Bio SEC 130 Å + Agilent Advance Bio SEC 300 Å (each: length 30 cm, diameter 4.6 mm, particle size 2.7 µm).
   Column oven temperature: 30 °C.
   Mobile phase: 0.15 M NaCl with bidest. H₂O.
   Flow rate 0.3 mL/min.
   Injection volume: 10 µL (12.3 mg/mL lyophilized dust in water).

Figure 3 shows a chromatogram of the stable dust Total extract that has been lyophilized and autoclaved.
Equipment and parameters:
Stable dust: Hechfellner Hof 2017.
System: Agilent Series 1100.
   Diode array detector: wavelength 210 nm.
   Columns: Agilent Advance Bio SEC 130 Å + Agilent Advance Bio SEC 300 Å (each: length 30 cm, diameter 4.6 mm, particle size 2.7 µm)
   Column oven temperature: 30 °C.
   Mobile phase: 0.15 M NaCl with bidest. H₂O.
   Flow rate 0.3 mL/min.
   Injection volume: 10 µL (9.9 mg/mL lyophilized dust in water).

Figure 5 shows a chromatogram of the > 10 kDa fraction of a stable dust extract that has not been lyophilized and not autoclaved.
Equipment and parameters:
Stable dust: Hechfellner Hof 2017.
System: Agilent Series 1100.
   Diode array detector: wavelength 210 nm.
   Columns: Agilent Advance Bio SEC 130 Å + Agilent Advance Bio SEC 300 Å (each: length 30 cm, diameter 4.6 mm, particle size 2.7 µm).
   Column oven temperature: 30 °C.
   Mobile phase: 0.15 M NaCl with bidest. H₂O.
   Flow rate 0.3 mL/min.
   Injection volume: 10 µL.

Figure 6 shows a chromatogram of the > 10 kDa fraction of a stable dust extract that has been lyophilized and not autoclaved.
Equipment and parameters:
Stable dust: Hechfellner Hof 2017.
System: Agilent Series 1100.
   Diode array detector: wavelength 210 nm.
   Columns: Agilent Advance Bio SEC 130 Å + Agilent Advance Bio SEC 300 Å (each: length 30 cm, diameter 4.6 mm, particle size 2.7 µm).
   Column oven temperature: 30 °C
   Mobile phase: 0.15 M NaCl with bidest. H₂O.
   Flow rate 0.3 mL/min.
   Injection volume: 10 µL (13.0 mg/mL lyophilized dust in water).

Figure 7 shows a chromatogram of the > 10 kDa fraction of a stable dust extract that has been lyophilized and autoclaved.
Equipment and parameters:
Stable dust: Hechfellner Hof 2017.
System: Agilent Series 1100.
   Diode array detector: wavelength 210 nm.
   Columns: Agilent Advance Bio SEC 130 Å + Agilent Advance Bio SEC 300 Å (each: length 30 cm, diameter 4.6 mm, particle size 2.7 µm).
   Column oven temperature: 30 °C.
   Mobile phase: 0.15 M NaCl with bidest. H₂O.
   Flow rate 0.3 mL/min.
   Injection volume: 10 µL (8.4 mg/mL lyophilized dust in water).

### Example 3: Change in chromatographic profile of a non-autoclaved and an autoclaved liquid extract over time under different storage conditions (room temperature, 4 °C and -20 °C).

Unless otherwise stated, barn dust extracts were prepared as described in Example 1. Ultracentrifugation step 9 was omitted.

Figure 9 illustrates the change in liquid extract (not autoclaved) measured after 8 days of storage under different conditions, namely storage at room temperature, refrigerated (storage at 4 °C) and freezed (storage at -20 °C)

Figure 10 illustrates the change in peak height for the peaks of respectively 27 kDA, 5 kDA, 1 kDa, and 0.6 kDa, measured after 8 days of storage under different conditions, namely storage at room temperature, storage at 4 °C and storage at -20 °C.

Figure 11 illustrates the difference between non-autoclaved and autoclaved liquid extracts obtained from barn dust from Hechfellner Hof.

Figure 12 illustrates the change in liquid extract (autoclaved) measured after 5 days of storage under different conditions, namely storage at room temperature, storage at 4 °C and storage at -20 °C.

Figure 13 illustrates the change in liquid extract (autoclaved) measured after 12 days of storage under different conditions, namely storage at room temperature, storage at 4 °C and storage at -20 °C.

Figure 14 illustrates the change in peak height for the peaks of respectively 309 kDa, 6 kDa, 2 kDa, and 0.8 kDa, measured after 8 days of storage under different conditions, namely storage at room temperature, storage at 4° and storage at -20°.

### Example 4: Measurement of eosinophil production in mice after intra-nasal administration of fractionated or unfractionated stable dust extracts

Figure 15 illustrates the experimental setup used to measure eosinophil recruitment to the lung in mice after intra-nasal administration of fractionated or unfractionated stable dust extracts.

Dust extracts (0.8 mg dry weight in 50 µL/treatment) are instilled intra-nasally every 2-3 days for a total of 8 times beginning at day 0 into 7-week old Balb/c mice that are sensitized intra-peritoneally with 50 µg of OVA-Alum (6 mg) at day 0 and 7, and challenged intra-nasally with 100 µg OVA at day 14, 15, and 16. Broncho-alveolar lavage (BAL) is performed at day 17 by delivering cold 1% BSA in PBS (2 mL) into the airway via a tracheal cannula and gently aspirating the fluid. Cells are counted using a Countess II FL automated cell counter (Thermo Fisher Scientific) and differentials are determined by an operator blinded to mouse ID/grouping after staining with Hema 3 (Thermo Fischer Scientific) (at least 400 cells/slide). Statistical significance for BAL cellularity measurements is determined by an unpaired two tailed Student's t-test. P-values < 0.05 are considered significant. Each treatment group includes a minimum of 5 mice

Figure 16 (left panel) illustrates eosinophils count after treating mice with a saline solution (negative control), an allergen (OVA, positive control), unfractionated, autoclaved stable dust extract, and unfractionated, non -autoclaved stable dust extract.

Figure 16 (right panel) illustrates eosinophils counts after treating mice with a saline solution (negative control), an allergen (OVA, positive control), unfractionated, autoclaved barn dust extract, fractions between 10 kDa and 100 kDa of autoclaved stable dust extract, and fractions bigger than 10 kDa of autoclaved stable dust extract.

### Example 5: Measurement of γδ T cell production in mice after intra-nasal administration of fractionated or unfractionated stable dust extracts

Mice are euthanized by lethal dose of anesthetic. Lungs are perfused and removed. To prepare single-cell suspension the lungs are minced with scissors and digested with 0.26 Wunsch U/mL of Liberase TM (Roche) and 4 U/ml of DNAse I (Sigma-Aldrich) at 37 °C for 1 h with shaking (60-70 rpm). The resulting suspension is passed through 20-22G needle and through the 70 µm cell strainer. After washing with complete medium, lung cells are resuspended in PBS/0.1% NaN₃/1.0% BSA at 5 × 10⁶ cells/mL. Cells are treated with Fc-Block for 10 min on ice and stained with fluorescently labeled antibodies to cell surface antigens for 30 min on ice in the dark. Cells are then washed and resuspended in PBS/0.1% NaN₃/1.0% BSA for flow cytometry.

The following antibodies to cell surface antigens are used: PE or BV510 conjugated hamster anti-mouse γδ T-Cell Receptor (GL3), FITC-hamster anti-mouse CD3ε (145-2C11), PE-Cy7 or PE-Cy5-rat anti-mouse CD4 (RM4-5), APC-R700-rat anti-mouse CD8α (53-6.7). All from BD Pharmingen. Flow cytometry is performed on a FACSCalibur or LSRII (BD Sciences) flow cytometers. Data is collected on 10000-20000 events. Data analysis is performed using CellQuest (BD) or FlowJo (FlowJo) software. Proportions of γδ T cells are reported as % of CD3+ T cells gating on lung lymphocytes.

Figure 18 illustrates the results of flow cytometry analysis to determine the proportion of CD3⁺γδ⁺ cells found in the lungs after treating mice with a saline solution (negative control). CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.

Figure 19 illustrates the results of flow cytometry analysis to determine the proportion of CD3⁺γδ⁺ cells found in the lungs after treating mice with an allergen (OVA). CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.

Figure 20 illustrates the results of flow cytometry analysis to determine the proportion of CD3⁺γδ⁺ cells found in the lungs after treating mice with allergen (OVA) + unfractionated, autoclaved barn dust extract. CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.

Figure 21 illustrates the results of flow cytometry analysis to determine the proportion of CD3⁺γδ⁺ cells found in the lungs after treating mice with allergen (OVA) + fractions of autoclaved stable dust extract between 10 kDa and 100 kDa. CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.

Figure 22 illustrates the results of flow cytometry analysis to determine the proportion of CD3⁺γδ⁺ cells found in the lungs after treating mice with allergen (OVA) + fractions of autoclaved stable dust extract > 10 kDa. CD3⁺γδ T cells are positive for both CD3 staining (FL1, vertical axis) and γδ staining (FL2, horizontal axis) and are located in the upper right quadrants of the two panels on the left. Each panel presents data from a different mouse (n=2). The two panels on the right present quantitative analyses for the corresponding panels on the left. Circled in red are the proportions of γδ T cells among CD3⁺ T cells found in each mouse.

## Claims

1. A method of preparing a barn dust extract, wherein the barn dust is from a cow barn, comprising:
(d) providing a mixture comprising a barn dust and a liquid;
(e) isolating a fraction of the mixture, wherein the barn dust extract comprised in the fraction consists essentially of molecules having a molecular weight of at least 10 kDa.

2. The method of claim 1, wherein the liquid is water, an aqueous solution, or a water-miscible solvent, preferably wherein the aqueous solution is a solution of sodium chloride, preferably normal saline.

3. The method of claim 1 or 2, wherein step (e) comprises enriching the fraction by factor of at least 5, preferably at least 10, preferably at least 20, preferably at least 100.

4. The method of any one of the preceding claims, further comprising the step of
(f) subjecting the mixture to heat treatment at a temperature of at least about 110 °C and/or a pressure of at least 1.5 bar for at least 3 min; and/or
(g) drying the fraction obtained in step (e).

5. The method of any one of the preceding claims, wherein the barn dust extract that consists essentially of molecules having a molecular weight of at least about 10 kDa comprises molecules having a molecular weight of at least about 100 kDa.

6. The method of any one of the preceding claims, wherein the barn dust extract essentially consists of water-soluble molecules.

7. A barn dust extract obtained by the method of any one of claims 1 to 6 or a composition comprising said barn dust extract.

8. The composition of claim 7, wherein the composition is a pharmaceutical composition.

9. The composition of claim 7 or 8, wherein the composition is in the form of a solution, an aerosol, a suspension, a lyophilisate, a powder, a tablet, a dragee, or a suppository.

10. The composition of any one of claims 7 to 9, wherein the composition is for nasal, inhaled, oral, conjunctival, subcutaneous, intraarticular, intraperitoneal, rectal, or vaginal administration.

11. The composition of any one of claims 7 to 10, wherein the composition is a food additive, a food ingredient, or a composition suitable to be distributed in indoor air.

12. The barn dust extract or the composition of any one of claims 7 to 11, for use in the prevention or treatment of an allergic disease selected from the group consisting of asthma and hay fever.

## Patentansprüche

1. Verfahren zur Herstellung eines Stallstaub-Extrakts, wobei der Stallstaub aus einem Kuhstall stammt, umfassend:
(d) Bereitstellen einer Mischung, die Stallstaub und eine Flüssigkeit umfasst;
(e) Isolieren einer Fraktion der Mischung, wobei der in der Fraktion enthaltene Stallstaub-Extrakt im Wesentlichen aus Molekülen mit einem Molekulargewicht von mindestens 10 kDa besteht.

2. Verfahren nach Anspruch 1, wobei die Flüssigkeit Wasser, eine wässrige Lösung oder ein mit Wasser mischbares Lösungsmittel ist, vorzugsweise wobei die wässrige Lösung eine Lösung von Natriumchlorid, vorzugsweise eine isotonische Kochsalzlösung, ist.

3. Verfahren nach Anspruch 1 oder 2, wobei Schritt (e) das Anreichern der Fraktion um einen Faktor von mindestens 5, vorzugsweise mindestens 10, vorzugsweise mindestens 20, vorzugsweise mindestens 100 umfasst.

4. Verfahren nach einem der vorstehenden Ansprüche, das ferner den Schritt umfasst,
(f) die Mischung einer Wärmebehandlung bei einer Temperatur von mindestens etwa 110 °C und/oder einem Druck von mindestens 1,5 bar für mindestens 3 min zu unterziehen; und/oder
(g) die in Schritt (e) erhaltene Fraktion zu trocknen.

5. Verfahren nach einem der vorstehenden Ansprüche, wobei der Stallstaub-Extrakt, der im Wesentlichen aus Molekülen mit einem Molekulargewicht von mindestens etwa 10 kDa besteht, Moleküle mit einem Molekulargewicht von mindestens etwa 100 kDa umfasst.

6. Verfahren nach einem der vorstehenden Ansprüche, wobei der Scheunenstaub-Extrakt im Wesentlichen aus wasserlöslichen Molekülen besteht.

7. Stallstaub-Extrakt, der nach einem der Verfahren gemäß den Ansprüchen 1 bis 6 hergestellt wurde, oder eine Zusammensetzung, die diesen Stallstaub-Extrakt enthält.

8. Zusammensetzung nach Anspruch 7, wobei die Zusammensetzung eine pharmazeutische Zusammensetzung ist.

9. Zusammensetzung nach Anspruch 7 oder 8, wobei die Zusammensetzung in Form einer Lösung, eines Aerosols, einer Suspension, eines Lyophilisats, eines Pulvers, einer Tablette, einer Dragee oder eines Zäpfchens vorliegt.

10. Zusammensetzung nach einem der Ansprüche 7 bis 9, wobei die Zusammensetzung zur nasalen, inhalativen, oralen, konjunktivalen, subkutanen, intraartikulären, intraperitonealen, rektalen oder vaginalen Verabreichung bestimmt ist.

11. Zusammensetzung nach einem der Ansprüche 7 bis 10, wobei die Zusammensetzung ein Lebensmittelzusatzstoff, ein Lebensmittelzutat oder eine Zusammensetzung ist, die zur Verteilung in der Raumluft geeignet ist.

12. Stallstaub-Extrakt oder die Zusammensetzung nach einem der Ansprüche 7 bis 11 zur Verwendung bei der Vorbeugung oder Behandlung einer allergischen Erkrankung, ausgewählt aus der Gruppe bestehend aus Asthma und Heuschnupfen.

## Revendications

1. Procédé de préparation d'un extrait de poussière d'étable, dans lequel la poussière d'étable provient d'une étable à vaches, comprenant :
(d) la fourniture d'un mélange comprenant une poussière d'étable et un liquide ;
(e) l'isolation d'une fraction du mélange, dans laquelle l'extrait de poussière d'étable compris dans la fraction est essentiellement constitué de molécules ayant un poids moléculaire d'au moins 10 kDa.

2. Procédé selon la revendication 1, dans lequel le liquide est de l'eau, une solution aqueuse ou un solvant miscible à l'eau, de préférence dans lequel la solution aqueuse est une solution de chlorure de sodium, de préférence une solution saline physiologique.

3. Procédé selon la revendication 1 ou 2, dans lequel l'étape (e) comprend l'enrichissement de la fraction d'un facteur d'au moins 5, de préférence d'au moins 10, de préférence d'au moins 20, de préférence d'au moins 100.

4. Procédé selon l'une quelconque des revendications précédentes, comprenant en outre l'étape consistant à
(f) soumettre le mélange à un traitement thermique à une température d'au moins environ 110 °C et/ou à une pression d'au moins 1,5 bar pendant au moins 3 minutes ; et/ou
(g) sécher la fraction obtenue à l'étape (e).

5. Procédé selon l'une quelconque des revendications précédentes, dans lequel l'extrait de poussière de grange qui consiste essentiellement en molécules ayant un poids moléculaire d'au moins environ 10 kDa comprend des molécules ayant un poids moléculaire d'au moins environ 100 kDa.

6. Procédé selon l'une quelconque des revendications précédentes, dans lequel l'extrait de poussière de grange est essentiellement constitué de molécules hydrosolubles.

7. Extrait de poussière de grange obtenu par le procédé selon l'une quelconque des revendications 1 à 6 ou composition comprenant ledit extrait de poussière de grange.

8. Composition selon la revendication 7, dans laquelle la composition est une composition pharmaceutique.

9. Composition selon la revendication 7 ou 8, dans laquelle la Composition se présente sous la forme d'une solution, d'un aérosol, d'une suspension, d'un lyophilisat, d'une poudre, d'un comprimé, d'un dragé ou d'un suppositoire.

10. Composition selon l'une quelconque des revendications 7 à 9, dans laquelle la composition est destinée à être administrée par voie nasale, inhalée, orale, conjonctivale, sous-cutanée, intra-articulaire, intra-péritonéale, rectale ou vaginale.

11. Composition selon l'une quelconque des revendications 7 à 10, dans laquelle la composition est un additif alimentaire, un ingrédient alimentaire ou une composition adaptée à être diffusée dans l'air intérieur.

12. Extrait de poussière de grange ou la composition de l'une quelconque des revendications 7 à 11, destinée à être utilisée dans la prévention ou le traitement d'une maladie allergique choisie dans le groupe constitué par l'asthme et le rhume des foins.
